# FASCICULE DE BREVET EUROPEEN

(11) **EP 0 436 449 B1**
(45) Date de publication et mention de la délivrance du brevet: **16.03.1994**
(21) Numéro de dépôt: 90420555.6
(22) Date de dépôt: 20.12.1990
(51) Int. Cl.: A61K 9/50, C08J 3/12, B01J 13/02

(54) **Microsphères magnétisables à base de polysilsesquioxane, leur procédé de préparation et leur application en biologie**
Magnetisierbare Polysilsesquioxanmikrokugeln, Verfahren zu ihrer Herstellung und ihre Verwendung in der Biologie
Magnetisable microspheres based on polysilsesquioxane, their preparation process and their biological use

(30) Priorité: 27.12.1989 FR 8917231
(43) Date de publication de la demande: 10.07.1991
(73) Titulaire: SOCIETE PROLABO, 75011 Paris (FR)
(72) Inventeur: Charmot, Dominique, F-75019 Paris (FR)
(74) Mandataire: Ahner, Francis

(56) Documents cités:
- EP-A- 0 125 995
- WO-A-78/00005
- WO-A-87/02063
- FR-A- 2 618 084
- FR-A- 2 624 873
- PATENT ABSTRACTS OF JAPAN vol. 13, no. 579 (C-668)(3927) 20 décembre 1989, & JP-A-1 242625 (TOSHIBA SILICONE CO., LTD.) 27 septembre 1989,

## Description

La présente invention a pour objet des microsphères magnétisables à base d'une matrice de polysilsesquioxane et de charges magnétisables uniformément réparties à l'intérieur de ladite matrice, le procédé de préparation desdites microsphères et leur application notamment en biologie.

La demanderesse a décrit dans sa demande de brevet français publiée sous le n° 2.618.084 des particules composites magnétisables constituées d'une matrice à base d'organopolysiloxane linéaire et, encapsulées dans ladite matrice, de charges magnétisables. La préparation de ce type de particules fait intervenir l'usage d'un fluide magnétique (ferrofluide) dont les charges magnétiques sont revêtues d'un agent dispersant rendu non hydrosoluble par décomposition thermique.

Sont également connues, d'après la demande de brevet français publiée sous le n°2.624.873, des particules magnétisables constituées d'une matrice dérivant de l'hydrosilylation d'un organopolysiloxane SiVi et d'un hydrogenopolysiloxane SiH et, encapsulés dans ladite matrice, de charges magnétisables revêtues d'un agent dispersant rendu non hydrosoluble par décomposition thermique.

La préparation de ces deux types de particules fait intervenir des charges magnétisables dont la surface est revêtue d'un agent dispersant rendu non hydrosoluble. La présence de cet agent dispersant peut être un inconvénient en biologie car ledit agent peut migrer vers la surface des particules et entraîner des phénomènes secondaires.

La demande européenne publiée sous le numéro 125.995 vise des particules dont la surface spécifique est au moins de 100m²/g, le diamètre est compris entre 0,1 et 1,5 µm, et présentant une structure "core - shell" ("coeur - écorce") dont le "coeur" est formé d'agrégats de particules et dont "l'écorce" est constituée par des adsorbants. Du type anticoprs, antigènes et proteines, ladite écorce étant liée au coeur au moyen d'un agent de couplage qui est un silane organofonctionnel.

La demanderesse a maintenant trouvé des microsphères magnétisables dont la charge magnétisable n'est pas recouverte d'un agent surfactant hydrophobe, ladite charge étant en outre répartie d'une manière homogène à l'intérieur de la matrice de silicone.

Selon l'invention il s'agit de microsphères magnétisables caractérisées en ce qu'elle sont constituées :
- d'une matrice à base d'un réseau de polysilsesquioxane ;
- et, réparties uniformément à l'intérieur dudit réseau, de charges magnétisables liées chimiquement à des motifs silsesquioxanes, lesdites charges présentant une taille généralement inférieure à 300 10⁻⁴ µm, de préférence de l'ordre de 50 à 120x10⁻⁴ µm ;
   lesdites microsphères présentant une surface spécifique BET de l'ordre de 2 à 50 m²/g, de préférence de l'ordre de 2 à 10 m²/g.

Les polysesquioxanes peuvent être obtenus par polycondensation d'un alcoxysilane de formule (I)

R-Si-(OR')₃ (I)

ou d'un alcoxysiloxane de formule (II) 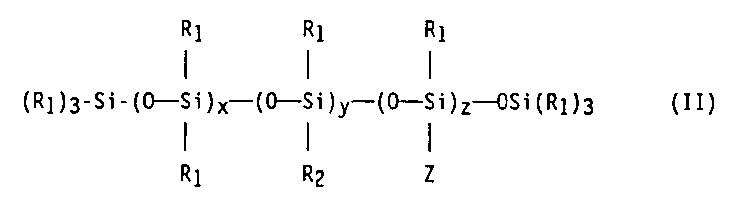
formules dans lesquelles
- R₁ représente :
   . un radical alkyle en C₁-C₃ (de préférence méthyle ou éthyle)
   . un radical phényle
- R₂ représente :
   . un radical R₁
   . un radical alkyle en C₁-C₄ substitué par des fonctions amino, époxy, mercapto, halogéno (de préférence aminopropyle, glycidylpropyle, mercatopropyle, bromopropyle, chloropropyle, trifluoropropyle)
   . un radical vinyle
- R représente :
   . un radical R₁
   . un radical R₂
   . un radical éthyleniquement insaturé (de préférence ester insaturé tel que méthacryloxy-propyle)
   . un radical diorganopolysiloxy présentant de 4 à 20 groupements diorganosiloxy
- OR' est un radical OH ou un radical hydrolysable tels que ceux dans lesquels R' représente :
   . un radical alkyle en C₁-C₄
   . un radical -CO-CH₃, -CO-C₂H₅, -CO-CH₂-CO-CH₃, -CH₂ CH₂OH, -CH₂CH₂ OCH₃, -CH₂-CH₂ OC₂H₅
- Z représente un radical -r-Si(R₁)-₃₋ₙ-(OR')ₙ dans lequel r est un groupement alkylène en C₁-C₁₈, de préférence en C₂-C₆ et n est un nombre entier de 0 à 3
- Les symboles x, y et z ont une valeur suffisante pour assurer une viscosité inférieure à 100 mPas à 25°C, les symboles x et y pouvant être séparément nuls.

Parmi les matériaux pouvant constituer les charges magnétisables, on peut citer la magnétite, l'hématite, le dioxyde de chrome, les ferrites telles que les ferrites de manganèse, de nickel, de manganèse-zinc.

Les matériaux préférentiels sont la magnétite et l'hématite. Ces matériaux peuvent également être présents en mélange avec des charges présentant un spectre de fluorescence, telles que oxyde ou oxysulfure d'yttrium activé à l'europium, borate de gadolinium-cérium-terbium, aluminate de cérium-terbium, aluminate de magnésium-baryum dopé à l'europium divalent.

La quantité de charges magnétisables correspond à environ 0,5 à 98 % (dont 0,01 % à 0,5 % des charges fluorescentes éventuelles) du poids des microsphères et de préférence de 5 à 80 % dudit poids.

Les microsphères magnétisables faisant l'objet de l'invention sont parfaitement sphériques ; elles peuvent être de taille uniforme ou présenter une granulométrie étalée ; leur diamètre peut être de l'ordre de 0,05 à 3 µm et généralement de l'ordre de 0,2 à 2 µm.

Elles peuvent se présenter telles quelles ou en dispersion dans l'eau ; la quantité de microsphères magnétisables à l'état dispersé dans l'eau peut correspondre à environ 10 à 70 % en poids par rapport au poids total de dispersion et généralement de l'ordre de 15 à 50 % en poids.

La présente invention a également pour objet le procédé de préparation des microsphères magnétisables ci-dessus décrites.

Ledit procédé consiste :
- à disperser dans un solvant organique non miscible à l'eau une suspension aqueuse de charges magnétisables non revêtues d'agent dispersant, charges présentant une taille généralement inférieure à 300x10⁻⁴µm, de préférence de l'ordre de 50x10⁻⁴ à 120x10⁻⁴ µm
- à dissoudre dans la phase organique de la dispersion obtenue un alcoxysilane de formule (I) ou un alcoxysiloxane de formule (II) susceptible de se polycondenser en un polysesquioxane
- à polycondenser ledit alcoxysilane ou alcoxysiloxane
- à éliminer l'eau
- à séparer les microsphères magnétisables
- et éventuellement à redisperser lesdites microsphères dans de l'eau.

Une variante du procédé consiste à introduire tout ou partie de l'alcoxysilane dans la suspension aqueuse de charges magnétisables préalablement à la mise en dispersion de ladite suspension aqueuse dans le solvant organique.

Le solvant organique mis en oeuvre à l'étape de dispersion est un solvant des alcoxysilanes ou alcoxysiloxanes de formules (I) ou (II). A titre d'exemples, on peut citer le cyclohexane, le chlorure de méthylène, le benzène, l'hexane, le toluène, le tétrachlorure de carbone, l'octane, les esters de diacides gras.

L'opération de dispersion est réalisée en une ou plusieurs étapes à une température de l'ordre de 20 à 60°C, à l'aide d'un système d'agitation énergique tel que moulin à colloïdes, pompes à haute pression, agitateur à vibration, appareil à ultra-sons.

La suspension aqueuse de charges magnétisables peut être obtenue par mise en suspension de charges broyées ; toutefois une forme préférentielle de suspension est un sol aqueux de charges magnétisables obtenu par tout procédé connu comme par exemple celui décrit dans le brevet US n° 3.480.555.

Le type de matériau pouvant constituer les charges à déjà été mentionné ci-dessus. D'autres charges peuvent être présentes à côté des charges magnétisables comme par exemple les charges luminescentes citées ci-dessus.

La concentration de charges magnétisables dans la suspension aqueuse peut être de l'ordre de 0,5 à 50 % en poids, généralement de l'ordre de 5 à 20 % en poids. La quantité de charges mise en oeuvre est telle que le rapport pondéral charges magnétisables/alcoxysilane ou alcoxysiloxane soit de l'ordre de 0,005 à 50.

La quantité de solvant organique mise en oeuvre est telle que le rapport pondéral phase aqueuse/phase organique soit de l'ordre de 0,005 à 2.

Un agent tensio-actif est mis en oeuvre pour réaliser l'opération de dispersion. Celui-ci peut être choisi parmi ceux permettant d'obtenir des émulsions eau dans l'huile (de HLB généralement inférieur à 10, de préférence inférieur à 5) tels que les agents non-ioniques du type esters d'acides gras du sorbitol, mono et trioleates de sorbitan, copolymères séquencés oxyde d'éthylène/oxyde de propylène, les alkylphenols ethoxylés contenant moins de 10 motifs éthoxylés, les polycondensats d'acides gras, les copolymères séquencés organosiloxane-oxyde d'éthylène-oxyde de propylène ; les agents anioniques tels que les dialkylsulfosuccinates ; les agents cationiques tels que le bromure de cétylammonium, les copolycondensats polyéthylèneimine-polyester.

L'opération de polycondensation est réalisée à une température de l'ordre de 20 à 80°C pendant environ 5 à 24 heures.

L'eau est ensuite éliminée par distillation par exemple.

Après refroidissement les microsphères magnétisables peuvent être séparées du milieu organique par tout moyen connu, notamment par aimantation.

Si désiré, lesdites microsphères magnétisables peuvent être redispersées dans de l'eau déionisée jusqu'à obtenir un taux d'extrait sec de l'ordre de 10 à 70 % en poids, de préférence de l'ordre de 15 à 50 % en poids. Cette opération est réalisée en présence d'au moins un agent tensio-actif permettant d'obtenir des emulsions huile dans l'eau (de HLB généralement supérieur à 10, de préférence supérieur à 15) tels que alkyl sufates, alkylsulfonates.

Les microsphères magnétisables faisant l'objet de l'invention sont notamment intéressantes en biologie.

Elles peuvent être utilisées par exemple comme supports actifs :
. d'anticorps ou d'antigènes pour les test de diagnostiques, les séparations par affinité des composés biologiques ; la fixation des molécules biologiques peut, si nécessaire, être réalisée par des méthodes de couplage bien connues faisant intervenir des agents de couplage (glutaraldéhyde, carbodiimide hydrosoluble), ou bien consistant à activer les fonctions éventuelles du polyorganosiloxane (par exemple par diazotation, par action du bromure de cyanogène, de l'hydrazine) et à faire réagir la molécule à fixer
. de systèmes enzymatiques pour réactions biologiques
. de fixation de cultures cellulaires
. de médicaments ou de substances de révélation pour guider ceux-ci ou celles-ci in vitro ou in vivo vers le point de traitement choisi
. de molécules chimiques permettant une croissance de ces molécules par enchaînement rapide de réactions particulières comme la synthèse peptidique
. de groupements chimiques catalyseurs de réaction
. de groupements chimiques pour la séparation ou l'extraction métaux ou d'isomères optiques.

Lesdites microsphères peuvent également être utilisées comme charge de renforcement pour élastomères ou pour la préparation de dispersions organiques utilisées dans les circuits hydrauliques de freins et d'amortisseurs.

Lorsqu'elles renferment en outre des charges luminescentes, elles peuvent être mises en oeuvre comme marqueur cellulaire ou comme agent de contraste en imagerie médicale.

Les exemples suivants sont donnés à titre indicatif.

La suspension aqueuse non surfactée d'oxyde de fer magnétique mise en oeuvre dans les exemples ci-après est préparée de la manière suivante.

175 g de Fe(NO3),9H20 et 75 g de Fe(SO4),7H20 sont dissous dans 250 g d'eau permutée et 55 g d'acide nitrique concentré ; sous agitation rapide on ajoute 250 g d'une solution aqueuse à 20 % d'ammoniaque. Après décantation et élimination de la solution surnageante, le précipité est lavé une fois par de l'eau. Le milieu est ensuite ajusté à pH 0,5 par 35 g d'acide perchlorique, et le précité filtré ; cette opération est répétée 3 fois, après quoi l'oxyde est remis en suspension dans de l'eau et ultrafiltré par de l'eau permutée. La suspension ainsi obtenue a une teneur en solide de 26,5 % pour un pH de 1,2. Le rendement exprimé en Fe304 est de 57 %. L'examen en microscopie electronique à transmission indique des tailles de particule d'oxyde de fer comprises entre 50x10⁻⁴ et 200x10⁻⁴ micromètres.

### EXEMPLE 1 :

### Préparation de microsphères magnétisables à base de poly(methyl)silsesquioxane

2 g de suspension d'oxyde de fer préparée ci-dessus sont dispersés dans un mélange constitué de 50 g de Solvesso 200® (coupe polyaromatique pétrolière fournie par Esso (France)) et 0,1 g de SPAN 80® (monooléate de sorbitan commercialisé par ICI (UK)), à l'aide d'un homogénéiseur ultrasonique. Cette émulsion inverse est chargée dans un réacteur en verre de 50 ml thermorégulé, muni d'une agitation mécanique et d'un condenseur. On introduit 2 g de méthyltriméthoxysilane (MTMS) et le milieu est laissé sous agitation à 20° C pendant 6 heures. L'eau présente dans l'émulsion est ensuite éliminée par distillation azéotropique. La teneur en solide de la suspension organique est 2,95 % en poids, soit un rendement de polycondensation proche de 100 % (exprimé en poids de poly(methyl)silsesquioxane formé).

La teneur en oxyde de fer dans les particules est de 35 %, estimée par dosage de fer en absorption atomique. Le pigment magnétique est réparti uniformément dans le volume de la bille comme l'atteste la vue donnée par microscopie électronique à transmission ; l'échelle mentionnée représente 0,09 micromètre ; les tailles de particules sont comprises entre 0,05 micromètre et 0,5 micromètre.

Ces mêmes particules sont collectées par aimantation et redispersées dans l'eau en présence de Cemulsol NP30® (nonyl-phénol-ehoxylé à 30 molécules d'oxyde d'éthylène, commercialisé par SFOS (France), à la concentration de 1 g/l pour former un latex magnétique à un extrait sec de 10 %.

### EXEMPLE 2 :

L'exemple 1 est répété à la différence que le Solvesso 200 est remplacé par du cyclohexane, et le SPAN 80® remplacé par l'Hypermer LP8 (agent dispersant commercialisé par ICI (UK)). Le rendement exprimé en poids de poly(methyl)silsesquioxane formé est proche de 100 %.

### EXEMPLE 3 :

L'exemple 1 est répété à la différence que le Solvesso® est remplacé par le Garbexol A6® (diester de l'acide adipique commercialisé par SFOS (France)). Le rendement exprimé en poids de poly(methyl)silsesquioxane formé est proche de 76 %.

### EXEMPLE 4 :

L'exemple 1 est répété en remplaçant le Solvesso 200® par de l'octane. Les 2 g de méthyltriméthoxysilane (MTMS) sont introduits au préalable dans la suspension d'oxyde de fer et agités quelques minutes pour hydrolyser le MTMS et obtenir une suspension homogène. Celle-ci est alors dispersée dans l'octane et homogénéisée aux ultra-sons. L'émulsion dans l'octane est agitée pendant une heure à 20° C puis on introduit 2 g supplémentaires de MTMS dans l'émulsion. Le traitement est poursuivi comme indiqué dans l'exemple 2. Le rendement exprimé en poids de poly(méthyl)silsesquioxane formé est proche de 100 %. La teneur en oxyde de fer dans les particules est de 21 %.

### EXEMPLE 5 :

### Préparation de microsphères magnétisables à base de Poly(vinyl)silsesquioxane

On reprend les conditions de l'exemple 1 en substituant le MTMS par du vinyl-triméthoxysilane (VTMO). Le rendement exprimé en poids de poly(vinyl) silsesquioxane formé est de 67 %. La teneur en oxyde de fer dans les particules est de 43 %.

### EXEMPLE 6 :

### Préparation de microsphères magnétisables à base de Poly(methacryl-oxypropyl)silsesquioxane

On reprend les conditions de l'exemple 1 en substituant le MTMS par du méthacryloxy-propyltriméthoxysilane (MEMO). Le rendement exprimé en poids de poly(methacryloxy-propyl)silsesquioxane formé est de 42 %. La teneur en oxyde de fer dans les particules est de 47 %.

### EXEMPLE 7 :

### Préparation de microsphères magnétisables à base de Poly(glycidyl-propyl)silsesquioxane

On reprend les conditions de l'exemple 1 en substituant le MTMS par du glycidyl-propyltriméthoxysilane (GLYMO). Le rendement exprimé en poids de poly(glycidyl-propyl)silsesquioxane formé est de 19 %. La teneur en oxyde de fer dans les particules est de 66 %.

### EXEMPLE 8 :

### Préparation de microsphères magnétisables à base de Poly(aminopropyl)silsesquioxane

On reprend les conditions de l'exemple 1 en substituant le MTMS par de l'amino-propyltriméthoxysilane (AMEO) et en l'introduisant au préalable dans la suspension aqueuse d'oxyde de fer ; ceci s'accompagne d'une floculation passagère de la suspension. Le reste du traitement est poursuivi comme indiqué dans l'exemple 1.

### EXEMPLE 9 : (comparatif à l'exemple 1)

Dans cet exemple on substitue la suspension aqueuse non surfactée d'oxyde de fer par une suspension d'oxyde de fer surfacté préparée selon le procédé revendiqué dans le brevet US 4094804 ; il s'agit d'oxyde de fer précipité en présence d'acide oleique, que l'on repeptise en milieu aqueux par ajout d'émulsifiant anionique (dioctylsulfosuccinate), Aérosol OT® commercialisé par American Cyanamid. La synthèse est poursuivie comme indiqué dans l'exemple 1 : dans ce cas on n'obtient pas de microsphères magnétisables ; en effet l'oxyde de fer a diffusé progressivement de la phase aqueuse vers la phase organique.

## Revendications (Revendications pour l'(les) Etat(s) contractant(s) suivant(s): AT, BE, CH, DE, DK, FR, GB, GR, IT, LI, LU, NL, SE)

1. Microsphères magnétisables caractérisées en ce qu'elle sont constituées :
- d'une matrice à base d'un réseau de polysilsesquioxane ;
- et, réparties uniformément à l'intérieur dudit réseau, de charges magnétisables liées chimiquement à des motifs silsesquioxanes, lesdites charges présentant une taille généralement inférieure à 300 10⁻⁴ µm, lesdites microsphères présentant une surface spécifique BET de l'ordre de 2 à 50 m²/g.

2. Microsphères selon la revendication 1 caractérisées en ce que les polysesquioxanes sont obtenus par polycondensation d'un alcoxysilane de formule (I)
R-Si-(OR')₃ (I)
ou d'un alcoxysiloxane de formule (II) 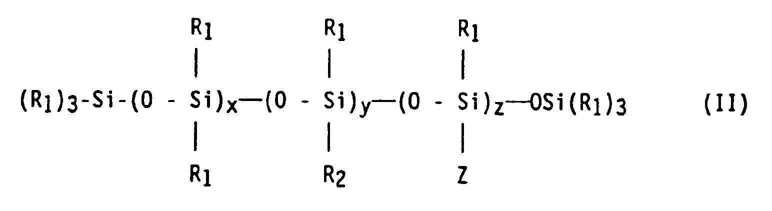 formules dans lesquelles
- R₁ représente :
. un radical alkyle en C₁-C₃ (de préférence méthyle ou éthyle)
. un radical phényle
- R₂ représente :
. un radical R₁
. un radical alkyle en C₁-C₄ substitué par des fonctions amino, époxy, mercapto, halogéno
. un radical vinyle
- R représente :
. un radical R₁
. un radical R₂
. un radical éthyléniquement insaturé (de préférence ester insaturé tel que méthacryloxy-propyle)
. un radical diorganopolysiloxy présentant de 4 à 20 groupements diorganosiloxy
- OR' est un radical OH ou un radical hydrolysable tels que ceux dans lesquels R' représente :
. un radical alkyle en C₁-C₄
. un radical -CO-CH₃, -CO-C₂H₅, -CO-CH₂-CO-CH₃, -CH₂-CH₂OH, -CH₂CH₂-OCH₃, -CH₂-CH₂-OC₂H₅
- Z représente un radical -r-Si(R₁)-₃₋ₙ-(OR')ₙ dans lequel r est un groupement alkylène en C₁-C₁₈, de préférence en C₂-C₆ et n est un nombre entier de 0 à 3
- Les symboles x, y et z ont une valeur suffisante pour assurer une viscosité inférieure à 100 mPas à 25°C, les symboles x et y pouvant être séparément nuls.

3. Microsphères selon la revendication 1 ou 2 caractérisées en ce que la quantité de charges magnétisables correspond à environ 0,5 à 98 % du poids des microsphères.

4. Microsphères selon l'une quelconque des revendications 1 à 3 caractérisées en ce que les charges magnétisables ont une taille de l'ordre de 50 à 120x10⁻⁴ micromètres.

5. Microsphères selon l'une quelconque des revendications 1 à 4 caractérisées en ce que leur diamètre est de l'ordre de 0,05 à 3 micromètres.

6. Procédé de préparation de microsphères magnétisables consistant :
- à disperser dans un solvant organique non miscible à l'eau une suspension aqueuse de charges magnétisables non revêtues d'agent dispersant, charges présentant une taille généralement inférieure à 300x10⁻⁴ µm
- à dissoudre dans la phase organique de la dispersion obtenue un alcoxysilane ou un alcoxysiloxane susceptible de se polycondenser en un polysesquioxane
- à polycondenser ledit alcoxysilane ou alcoxysiloxane
- à éliminer l'eau
- à séparer les microsphères magnétisables
- et éventuellement à redisperser lesdites microsphères dans de l'eau.

7. Procédé selon la revendication 6 caractérisé en ce que l'alcoxysilane a pour formule (I) :
R-Si-(OR')₃ (I)
et l'alcoxysiloxane a pour formule (II) : 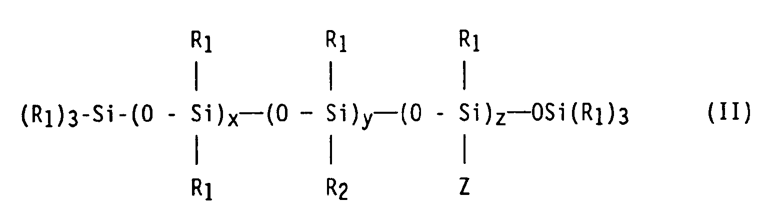 formules dans lesquelles
- R₁ représente :
. un radical alkyle en C₁-C₃
. un radical phényle
- R₂ représente :
. un radical R₁
. un radical alkyle en C₁-C₄ substitué par des fonctions amino, époxy, mercapto, halogéno
. un radical vinyle
- R représente :
. un radical R₁
. un radical R₂
. un radical éthyléniquement insaturé tel que méthacryloxy-propyle
. un radical diorganopolysiloxy présentant de 4 à 20 groupements diorganosiloxy
- OR' est un radical OH ou un radical hydrolysable tels que ceux dans lesquels R' représente
. un radical alkyle en C₁-C₄
. un radical -CO-CH₃, -CO-C₂H₅, -CO-CH₂-CO-CH₃, -CH₂-CH₂OH, -CH₂CH₂-OCH₃, -CH₂-CH₂-OC₂H₅
- Z représente un radical -r-Si(R₁)₃₋ₙ-(OR')ₙ dans lequel r est un groupement alkylène en C₁-C₁₈, de préférence en C₂-C₆ et n est un nombre entier de 0 à 3
- Les symboles x, y et z ont une valeur suffisante pour assurer une viscosité inférieure à 100 mPas à 25°C, les symboles x et y pouvant être séparement nuls.

8. Procédé selon l'une quelconque des revendications 6 ou 7 caractérisé en ce que les charges magnétisables présentent une taille de l'ordre de 50 à 120x10⁻⁴ micromètres.

9. Procédé selon l'une quelconque des revendications 6 à 8 caractérisé en ce que la concentration de charges magnétisables dans la suspension aqueuse est de l'ordre de 0,5 à 50 % en poids et en ce que la quantité de charges mise en oeuvre est telle que le rapport pondéral charges magnétisables/alcoxysilane ou alcoxysiloxane soit de l'ordre de 0,005 à 50.

10. Procédé selon l'une quelconque des revendications 6 à 9 caractérisé en ce que la quantité de solvant organique mise en oeuvre est telle que le rapport pondéral phase aqueuse/phase organique soit de l'ordre de 0,005 à 2.

11. Utilisation des microsphères telles quelles ou en dispersion aqueuse faisant l'objet de l'une quelconque des revendications 1 à 5 ou obtenues selon le procédé faisant l'objet de l'une quelconque des revendications 6 à 10 comme supports actifs en biologie, à condition que les méthodes de traitement thérapeutique telles que définies à l'article 52(4) C.B.E. soient exclues.

## Revendications (Revendications pour l'(les) Etat(s) contractant(s) suivant(s): ES)

1. Procédé de préparation de microsphères magnétisables, caractérisées en ce qu'elles sont constituées :
- d'une matrice à base d'un réseau de polysilsesquioxane ;
- et, réparties uniformément à l'intérieur dudit réseau, de charges magnétisables liées chimiquement à des motifs silsesquioxanes, lesdites charges présentant une taille généralement inférieure à 300 10⁻⁴ µm, lesdites microsphères présentant une surface spécifique BET de l'ordre de 2 à 50 m²/g,
ledit procédé consistant :
- à disperser dans un solvant organique non miscible à l'eau une suspension aqueuse de charges magnétisables non revêtues d'agent dispersant, charges présentant une taille généralement inférieure à 300x10⁻⁴ µm
- à dissoudre dans la phase organique de la dispersion obtenue un alcoxysilane ou un alcoxysiloxane susceptible de se polycondenser en un polysesquioxane
- à polycondenser ledit alcoxysilane ou alcoxysiloxane
- à éliminer l'eau
- à séparer les microsphères magnétisables
- et éventuellement à redisperser lesdites microsphères dans de l'eau.

2. Procédé selon la revendication 1 caractérisé en ce que les polysesquioxanes sont obtenus par polycondensation d'un alcoxysilane de formule (I)
R-Si-(OR')₃ (I)
ou d'un alcoxysiloxane de formule (II) 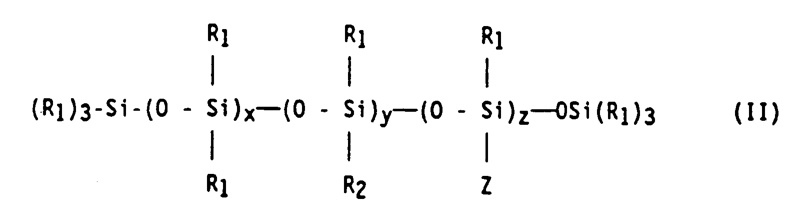 formules dans lesquelles
- R₁ représente :
. un radical alkyle en C₁-C₃ (de préférence méthyle ou éthyle)
. un radical phényle
- R₂ représente :
. un radical R₁
. un radical alkyle en C₁-C₄ substitué par des fonctions amino, époxy, mercapto, halogéno
. un radical vinyle
- R représente :
. un radical R₁
. un radical R2
. un radical éthyléniquement insaturé (de préférence ester insaturé tel que méthacryloxy-propyle)
. un radical diorganopolysiloxy présentant de 4 à 20 groupements diorganosiloxy
- OR' est un radical OH ou un radical hydrolysable tels que ceux dans lesquels R' représente :
. un radical alkyle en C₁-C₄
. un radical CO-CH₃, -CO-C₂H₅, -CO-CH₂-CO-CH₃, -CH₂-CH₂OH, -CH₂CH₂-OCH₃, -CH₂-CH₂-OC₂H₅
- Z représente un radical -r-Si(R₁)-₃₋ₙ-(OR')ₙ dans lequel r est un groupement alkylène en C₁-C₁₈, de préférence en C₂-C₆ et n est un nombre entier de 0 à 3
- Les symboles x, y et z ont une valeur suffisante pour assurer une viscosité inférieure à 100 mPas à 25°C, les symboles x et y pouvant être séparément nuls.

3. Procédé selon la revendication 1 ou 2 caractérisé en ce que la quantité de charges magnétisables correspond à environ 0,5 à 98 % du poids des microsphères.

4. Procédé selon l'une quelconque des revendications 1 à 3 caractérisé en ce que les charges magnétisables ont une taille de l'ordre de 50 à 120x10⁻⁴ micromètres.

5. Procédé selon l'une quelconque des revendications 1 à 4 caractérisé en ce que leur diamètre est de l'ordre de 0,05 à 3 micromètres.

6. Procédé selon l'une quelconque des revendications 1 à 5 caractérisé en ce que la concentration de charges magnétisables dans la suspension aqueuse est de l'ordre de 0,5 à 50 % en poids et en ce que la quantité de charges mise en oeuvre est telle que le rapport pondéral charges magnétisables/alcoxysilane ou alcoxysiloxane soit de l'ordre de 0,005 à 50.

7. Procédé selon l'une quelconque des revendications 1 à 6 caractérisé en ce que la quantité de solvant organique mise en oeuvre est telle que le rapport pondéral phase aqueuse/phase organique soit de l'ordre de 0,005 à 2.

8. Utilisation des microsphères telles quelles ou en dispersion aqueuse faisant l'objet de l'une quelconque des revendication 1 à 7 comme supports actifs en biologie, à condition que les méthodes de traitement thérapeutique telles que définies à l'article 52(4) CBE soient exclues.

## Claims (Claims for the following Contracting State(s): AT, BE, CH, DE, DK, FR, GB, GR, IT, LI, LU, NL, SE)

1. Magnetizable microspheres characterized in that they consist of:
- a matrix based on a polysilsesquioxane skeleton;
- and, uniformly distributed inside the said skeleton, magnetizable fillers chemically bonded to silsesquioxane units, the said fillers having a size generally smaller than 300 10⁻⁴ µm, the said microspheres having a BET specific surface area of the order of 2 to 50 m²/g.

2. Microspheres according to claim 1, characterized in that the polysilsesquioxanes are obtained by polycondensation of an alkoxysilane of formula (I)
R Si (OR')₃ (I)
or of an alkoxysiloxane of formula (II) 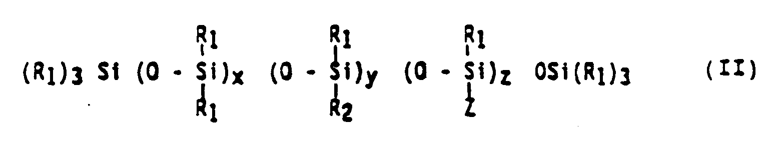 in which formulae
- R₁ represents
. a C₁-C₃ alkyl radical or
. a phenyl radical,
- R₂ represents
. a radical R₁,
. a C₁-C₄ alkyl radical substituted by amino, epoxy, mercapto, halogeno functions or
. a vinyl radical,
- R represents
. a radical R₁,
. a radical R₂,
. an ethylenically unsaturated radical(preferably unsaturated ester such as methacryloxypropyl),
. a diorganopolysiloxy radical having from 4 to 20 diorganosiloxy groups,
- OR' is an OH radical or a hydrolysable radical such as those in which R' represents
. a C₁-C₄ alkyl radical or
. a -CO-CH₃, -CO-C₂H₅, -CO-CH₂-CO-CH₃, -CH₂ CH₂OH, -CH₂CH₂ OCH₃, -CH₂-CH₂ OC₂H₅ radical,
- Z represents a radical -r-Si(R₁)₃₋ₙ (OR')ₙ, in which r is a C₁-C₁₈, preferably C₂-C₆, alkylene group, and
- the symbols x, y and z have a value sufficient to ensure a viscosity lower than 100 mPas at 25°C, it being possible for the symbols x and y separately to be zero.

3. Microspheres according to claim 1 or 2, characterized in that the amount of magnetizable fillers corresponds to about 0.5 to 98 % of the weight of the microspheres.

4. Microspheres according to any one of claims 1 to 3, characterized in that the magnetizable fillers have a size of the order of 50 to 120 10⁻⁴ microns.

5. Microspheres according to any one of claims 1 to 4, characterized in that their diameter is of the order of 0.05 to 3 microns.

6. Process for the preparation of magnetizable microspheres, consisting in:
- dispersing an aqueous suspension of magnetizable fillers not coated with dispersing agent, said fillers having a size generally smaller than 300 10⁻⁴ µm, in an organic solvent immiscible with water,
- dissolving an alkosysilane or alkoxysiloxane, capable of undergoing polycondensation to form a polysesquioxane, in the organic phase of the dispersion obtained,
- subjecting the said alcoxysilane or alcoxysiloxane to polycondensation,
- removing the water,
- separating off the magnetizable microspheres
- and, optionally, redispersing the said microspheres in water.

7. Process according to claim 6, characterized in that the alkoxysilane has the formula (I)
R Si (OR')₃ (I)
and the alkoxysiloxane has the formula (II) 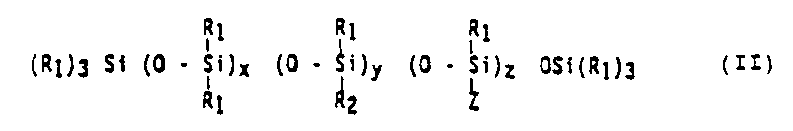 in which formulae
- R₁ represents
. a C₁-C₃ alkyl radical or
. a phenyl radical,
- R₂ represents
. a radical R₁,
. a C₁-C₄ alkyl radical substituted by amino, epoxy, mercapto, halogeno functions or
. a vinyl radical,
- R represents
. a radical R₁,
. a radical R₂,
. an ethylenically unsaturated radical such as methacryloxypropyl,
. a diorganopolysiloxy radical having from 4 to 20 diorganosiloxy groups,
- OR' is an OH radical or a hydrolysable radical such as those in which R' represents
. a C₁-C₄ alkyl radical or
. a -CO-CH₃, -CO-C₂H₅, -CO-CH₂-CO-CH₃, -CH₂ CH₂OH, -CH₂CH₂ OCH₃, -CH₂-CH₂, OC₂H₅ radical,
- Z represents a radical -r-Si(R₁)₃₋ₙ (OR')ₙ, in which r is a C₁-C₁₈, preferably C₂-C₆, alkylene group, and
- the symbols x, y and z have a value sufficient to ensure a viscosity lower than 100 mPas at 25°C, it being possible for the symbols x and y separately to be zero.

8. Process according to any one of claims 6 or 7, characterized in that the magnetizable fillers have a size of the order of 50 to 120 10⁻⁴ microns.

9. Process according to any one of claims 6 to 8, characterized in that the concentration of magnetizable fillers in the aqueous suspension is of the order of 0.5 to 50 % by weight and in that the amount of fillers used is such that the ratio, by weight, of magnetisable fillers alkoxysilane or alcoxysiloxane is of the order of 0.005 to 50.

10. Process according to any one of claims 6 to 9, characterized in that the amount of organic solvent used is such that the ratio, by weight, of aqueous phase/organic phase is of the order of 0.005 to 2.

11. Use of microspheres, as such or in aqueous dispersion, which are the subject of any one of claims 1 to 5 or are obtained by the process which is the subject of any one of claims 6 to 10 as active supports in biology, proviso that the therapeutic methods of treatments such as defined in article 52(4) EPC be disclaimed.

## Claims (Claims for the following Contracting State(s): ES)

1. Process of preparation of microspheres characterized in that they consist of:
- a matrix based on a polysilsesquioxane skeleton;
- and, uniformly distributed inside the said skeleton, magnetisable fillers chemically bonded to silsesquioxane units, the said fillers having a size generally smaller than 300 10⁻⁴ µm, the said microspheres having a BET specific surface area of the order of 2 to 50 m²/g , said process consisting in:
- dispersing an aqueous suspension of magnetizable fillers not coated with dispersing agent, said fillers having a size generally smaller than 300 10⁻⁴ µm, in an organic solvent immiscible with water,
- dissolving an alkoxysilane or an alkoxysiloxane, capable of undergoing polycondensation to form a polysesquioxane, in the organic phase of the dispersion obtained,
- subjecting the said alkoxysilane or alkoxysiloxane to polycondensation,
- removing the water,
- separating off the magnetizable microspheres
- and, optionally, redispersing the said microspheres in water.

2. Process according to claim 1, characterized in that the polysilsesquioxanes are obtained by polycondensation of an alkoxysilane of formula (I)
R Si (OR')₃ (I)
or of an alkoxysiloxane of formula (II) 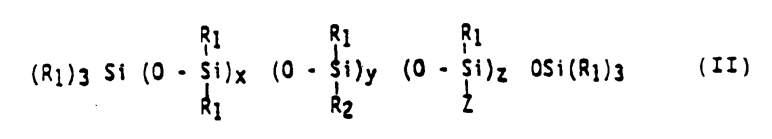 in which formualae
- R₁ represents
. a C₁-C₃ alkyl radical preferably methyle or ethyle or
. a phenyl radical,
- R₂ represents
. a radical R₁,
. a C₁-C₄ alkyl radical substituted by amino, epoxy, mercapto, halogeno functions or
. a vinyl radical,
- R represents
. a radical R₁,
. a radical R₂,
. an ethylenically unsaturated radical (preferably unsaturated ester such as methacryloxypropyl),
. a diorganopolysiloxy radical having from 4 to 20 diorganosiloxy grougs,
- OR' is an OH radical or a hydrolysable radical such as those in which R' represents
. a C₁-C₄ alkyl radical or
. a -CO-CH₃, -CO-C₂H₅, -CO-CH₂-CO-CH₃, -CH₂ CH₂OH, -CH₂CH₂ OCH₃, -CH₂-CH₂ OC₂H₅ radical,
- Z represents a radical -r-Si(R₁)₃₋ₙ (OR')ₙ, in which r is a C₁-C₁₈, preferably C₂-C₆, alkylene group, and n is an integral number from 0 to 3,
- the symbols x, y and z have a value sufficient to ensure a viscosity lower than 100 mPas at 25°C, it being possible for the symbols x and y separately to be zero.

3. Process according to claim 1 or 2, characterized in that the amount of magnetizable fillers corresponds to about 0.5 to 98 % of the weight of the microspheres.

4. Process according to any one of claims 1 to 3, characterized in that the magnetizable fillers have a size of the order of 50 to 120 10⁻⁴ microns.

5. Process according to any one of claims 1 to 4, characterized in that their diameter is of the order of 0.05 to 3 microns.

6. Process according to any one of claims 1 to 5, characterized in that the concentration of magnetizable fillers in the aqueous suspension is of the order of 0.5 to 50 % by weight and in that the amount of fillers used is such that the ratio, by weight, of magnetizable fillers/alkoxysilane or alkoxysiloxane is of the order of 0.005 to 50.

7. Process acording to any one of claims 1 to 6, characterized in that the amount of organic solvent used is such that the ratio, by weight, of aqueous phase/organic phase is of the order of 0.005 to 2.

8. Use of microspheres, as such or in aqueous dispersion, which are the subject of any one of claims 1 to 7 as active supports in biology proviso that the therapeutic methods of treatment such as defined in article 52 (4) EPC be disclaimed.

## Patentansprüche (Patentansprüche für folgende(n) Vertragsstaat(en): AT, BE, CH, DE, DK, FR, GB, GR, IT, LI, LU, NL, SE)

1. Magnetisierbare Mikrokugeln, dadurch gekennzeichnet, daß sie bestehen aus:
- einer Matrix auf der Basis eines Polysilasesquioxansharzes;
- und im Inneren des genannten Harzes gleichmäßig verteilten magnetisierbaren Füllungen, chemisch verbunden mit den Silasesquioxangruppen, wobei die genannten Füllungen im allgemeinen eine Größe kleiner als 300 10⁻⁴ µm aufweisen, und wobei die genannten Mikrokugeln eine spezifische BET-Oberfläche im Bereich von 2 bis 50 m²/g aufweisen.

2. Mikrokugeln nach Anspruch 1, dadurch gekennzeichnet, daß die Polysesquioxane erhalten werden durch Polykondensation eines Alkoxysilans mit der Formel (I)
R-Si-(OR')₃ (I)
oder eines Alkoxysiloxans mit der Formel (II) 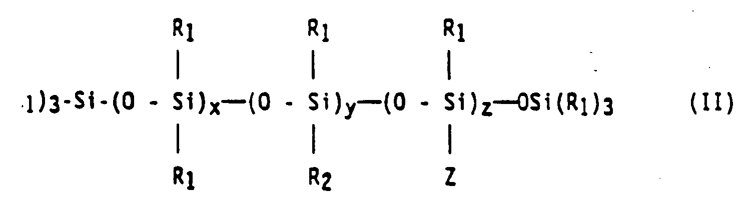 wobei in der Formel
- R₁ bedeutet:
. einen C₁-C₃-Alkylrest (bevorzugt Methyl oder Ethyl)
. einen Phenylrest
- R₂ bedeutet:
. einen R₁-Rest
. einen C₁-C₄-Alkylrest, substituiert mit Amino-, Epoxy-, Mercapto-, Halogenfunktionen
. einen Vinylrest
- R bedeutet:
. einen Rest R₁
. einen Rest R₂
. einen ethylenisch ungesättigten Rest (bevorzugt einen ungesättigten Ester wie Methacryloxy-Propyl)
. einen Diorganopolysiloxyrest, der 4 bis 20 Diorganosiloxygruppen aufweist
- OR' einen OH-Rest oder einen hydrolysierbaren Rest bedeutet wie solche, worin R' bedeutet:
. einen C₁-C₄-Alkylrest
. einen -CO-CH₃-, -CO-C₂H₅-, -CO-CH₂-CO-CH₃-, -CH₂-CH₂OH-, -CH₂CH₂-OCH₃-, -CH₂-CH₂-OC₂H₅-Rest
- Z einen -r-Si(R₁)-₃₋ₙ-(OR' )ₙ-Rest bedeutet, worin r eine C₁-C₁₈-Alkylengruppe, bevorzugt C₂-C₆, ist, und n eine ganze Zahl von 0 bis 3 ist;
- die Symbole x, y, z einen ausreichenden Wert haben, um eine Viskosität niedriger als 100 mPas bei 25°C zu gewährleisten, wobei die Symbole x und y jeweils unabhängig voneinander Null sein können.

3. Mikrokugeln nach Anspruch 1 oder 2, dadurch gekennzeichnet, daß die Menge der magnetisierbaren Füllungen etwa 0,5 bis 98 Gew.% der Mikrokugeln entspricht.

4. Mikrokugeln nach einem der Ansprüche 1 bis 3, dadurch gekennzeichnet, daß die magnetisierbaren Füllungen eine Größe in dem Bereich von 50 bis 120x10⁻⁴ Mikrometern haben.

5. Mikrokugeln nach einem der Ansprüche 1 bis 4, dadurch gekennzeichnet, daß ihr Durchmesser in dem Bereich von 0,05 bis 3 Mikrometern liegt.

6. Verfahren zur Herstellung von magnetisierbaren Mikrokugeln, welches besteht aus:
- Dispergieren einer wässrigen Suspension von magnetisierbaren, nicht mit Dispergiermittel umhüllten Füllungen in einem organischen, nicht mit Wasser mischbaren Lösungsmittel, wobei die Füllungen im allgemeinen eine Größe kleiner als 300x10⁻⁴ µm aufweisen
- Lösen eines Alkoxysilans oder eines zu einem Polysesquioxan polykondensierbaren Alkoxysiloxans in der organischen Phase der erhaltenen Suspension
- Polykondensieren des Alkoxysilans oder Alkoxysiloxans
- Eliminieren von Wasser
- Abtrennen der magnetisierbaren Mikrokugeln
- und gegebenenfalls erneutem Dispergieren der genannten Mikrokugeln in Wasser.

7. Verfahren nach Anspruch 6, dadurch gekennzeichnet, daß das Alkoxysilan die Formel (I) hat:
R-Si-(OR')₃
und das Alkoxysiloxan die Formel (II) hat: 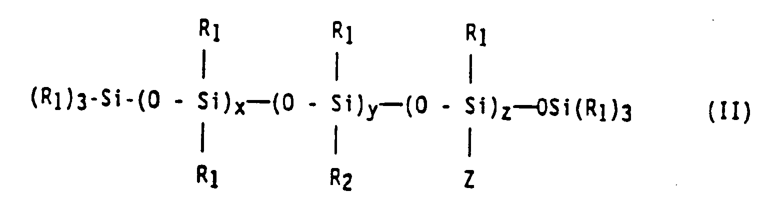 wobei in den Formeln
- R₁ bedeutet:
. einen C₁-C₃-Alkylrest
. einen Phenylrest
- R₂ bedeutet:
. einen R₁-Rest
. einen C₁-C₄-Alkylrest, substituiert mit Amino-, Epoxy-, Mercapto-, Halogenfunktionen
. einen Vinylrest
- R bedeutet:
. einen Rest R₁
. einen Rest R₂
. einen ethylenisch ungesättigten Rest wie Methacryloxy-Propyl
. einen Diorganopolysiloxyrest, der 4 bis 20 Diorganosiloxygruppen aufweist
- OR' einen OH-Rest oder einen hydrodysierbaren Rest bedeutet wie solche, worin R' bedeutet:
. einen C₁-C₄-Alkylrest
. einen -CO-CH₃-, -CO-C₂H₅-, -CO-CH₂-CO-CH₃-, -CH₂-CH₂OH-, -CH₂CH₂-OCH₃-, -CH₂-CH₂-OC₂H₅-Rest
- Z einen -r-Si(R₁)-₃₋ₙ(OR')ₙ-Rest bedeutet, worin r eine C₁-C₁₈-Alkylengruppe, bevorzugt C₂-C₆, ist, und n eine ganze Zahl von 0 bis 3 ist
- die Symbole x, y, z einen ausreichenden Wert haben, um eine Viskosität niedriger als 100 mPas bei 25°C zu gewährleisten, wobei die Symbole x und y jeweils unabhängig voneinander Null sein können.

8. Verfahren nach einem der Ansprüche 6 oder 7, dadurch gekennzeichnet, daß die magnetisierbaren Füllungen eine Größe in dem Bereich von 50 bis 120x10⁻⁴ Mikrometern aufweisen.

9. Verfahren nach einem der Ansprüche 6 bis 8, dadurch gekennzeichnet, daß die Konzentration der magnetisierbaren Füllungen in der wässrigen Suspension im Bereich von 0,5 bis 50 Gew.% liegt und daß die Menge der verwendeten Füllungen so ist, daß das Gewichtsverhältnis magnetisierbarer Füllungen/Alkoxysilan oder Alkoxysiloxan in dem Bereich von 0,005 bis 50 liegt.

10. Verfahren nach einem der Ansprüche 6 bis 9, dadurch gekennzeichnet, daß die Menge des verwendeten organischen Lösungsmittels so ist, daß das Gewichtsverhältnis wässrige Phase/organische Phase in dem Bereich von 0,005 bis 2 liegt.

11. Verwendung der Mikrokugeln, die Gegenstand von eines der Ansprüche 1 bis 5 sind oder erhalten werden nach dem Gegenstand eines der Ansprüche 6 bis 10 als solche oder in wässriger Dispersion, als biologisch aktive Träger, mit der Maßgabe, daß die in Artikel 52 (4) EPÜ definierten therapeutischen Behandlungsmethoden ausgeschlossen sind.

## Patentansprüche (Patentansprüche für folgende(n) Vertragsstaat(en): ES)

1. Verfahren zur Herstellung von magnetisierbaren Mikrokugeln, dadurch gekennzeichnet, daß sie bestehen aus:
- einer Matrix auf der Basis eines Polysilasesquioxansharzes;
- und im Inneren des genannten Harzes gleichmäßig verteilten magnetisierbaren Füllungen, chemisch verbunden mit den Silasesquioxangruppen, wobei die genannten Füllungen im allgemeinen eine Größe kleiner als 300 10⁻⁴ µm aufweisen, und wobei die genannten Mikrokugeln eine spezifische BET-Oberfläche in dem Bereich von 2 bis 50 m²/g aufweisen,
wobei das genannte Verfahren besteht aus:
- Dispergieren einer wässrigen Suspension von magnetisierbaren, nicht mit Dispergiermittel umhüllten Füllungen in einem organischen, nicht mit Wasser mischbaren Lösungsmittel, wobei die Füllungen im allgemeinen eine Größe kleiner als 300x10⁻⁴ µm aufweisen
- Lösen eines Alkoxysilans oder eines zu einem Polysesquioxan polykondensierbaren Alkoxysiloxans in der organischen Phase der erhaltenen Suspension
- Polykondensieren des Alkoxysilans oder Alkoxysiloxans
- Eliminieren von Wasser
- Abtrennen der magnetisierbaren Mikrokugeln
- und gegebenenfalls erneuten Dispergieren der genannten Mikrokugeln in Wasser.

2. Verfahren nach Anspruch 1, dadurch gekennzeichnet, daß die Polysesquioxane erhalten werden durch Polykondensation eines Alkoxysilans mit der Formel (I)
R-Si-(OR')₃ (I)
oder eines Alkoxysiloxans mit der Formel (II) 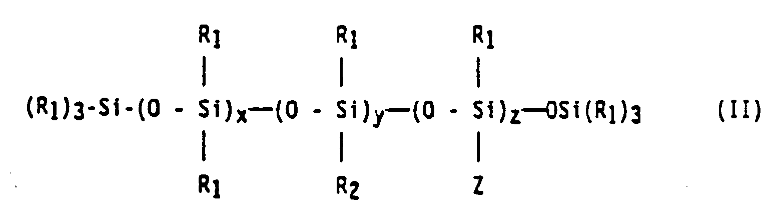 wobei in der Formel
- R₁ bedeutet:
. einen C₁-C₃-Alkylrest (bevorzugt Methyl oder Ethyl)
. einen Phenylrest
- R₂ bedeutet:
. einen R₁-Rest
. einen C₁-C₄-Alkylrest, substituiert mit Amino-, Epoxy-, Mercapto-, Halogenfunktionen
. einen Vinylrest
- R bedeutet:
. einen Rest R₁
. einen Rest R₂
. einen ethylenisch ungesättigten Rest (bevorzugt einen ungesättigten Ester wie Methacryloxy-Propyl)
. einen Diorganopolysiloxyrest, der 4 bis 20 Diorganosiloxygruppen aufweist
- OR' einen OH-Rest oder einen hydrolysierbaren Rest bedeutet wie solche, worin R' bedeutet:
. einen C₁-C₄-Alkylrest
. einen -CO-CH₃-, -CO-C₂H₅-, -CO-CH₂-CO-CH₃-, -CH₂-CH₂OH-, -CH₂CH₂-OCH₃-, -CH₂-CH₂-OC₂H₅-Rest
- Z einen -r-Si(R₁)-₃₋ₙ-(OR')ₙ-Rest bedeutet, worin r eine C₁-C₁₈-Alkylengruppe, bevorzugt C₂-C₆, ist, und n eine ganze Zahl von 0 bis 3 ist;
- die Symbole x, y, z einen ausreichenden Wert haben, um eine Viskosität niedriger als 100 mPas bei 25°C zu gewährleisten, wobei die Symbole x und y jeweils unabhängig von einander Null sein können.

3. Verfahren nach Anspruch 1 oder 2, dadurch gekennzeichnet, daß die Menge der magnetisierbaren Füllungen etwa 0,5 bis 98 Gew.% der Mikrokugeln entspricht.

4. Verfahren nach einem der Ansprüche 1 bis 3, dadurch gekennzeichnet, daß die magnetisierbaren Füllungen eine Größe im Bereich von 50 bis 120x10⁻⁴ Mikrometern haben.

5. Verfahren nach einem der Ansprüche 1 bis 4, dadurch gekennzeichnet, daß ihr Durchmesser im Bereich von 0,05 bis 3 Mikrometern liegt.

6. Verfahren nach einem der Ansprüche 1 bis 5, dadurch gekennzeichnet, daß die Konzentration der magnetisierbaren Füllungen in der wässrigen Suspension in dem Bereich von 0,5 bis 50 Gew.% liegt und daß die Menge der verwendeten Füllungen so ist, daß das Gewichtsverhältnis magnetisierbarer Füllungen/Alkoxysilan oder Alkoxysiloxan in dem Bereich von 0,005 bis 50 liegt.

7. Verfahren nach einem der Ansprüche 1 bis 6, dadurch gekennzeichnet, daß die Menge des verwendeten organischen Lösungsmittels so ist, daß das Gewichtsverhältnis wässrige Phase/organische Phase in dem Bereich von 0,005 bis 2 liegt.

8. Verwendung der Mikrokugeln, die Gegenstand eines der Ansprüche 1 bis 7 sind, als solche oder in wässriger Dispersion, als biologisch aktive Träger, mit der Maßgabe, daß die in Artikel 52 (4) EPÜ definierten therapeutischen Behandlungsmethoden ausgeschlossen sind.
